(19) Europäisches Patentamt
European Patent Office
Office européen des brevets

(11) EP 2 960 246 A1

# (12) EUROPEAN PATENT APPLICATION

(43) Date of publication:
**30.12.2015 Bulletin 2015/53**

(21) Application number: **15171792.3**

(22) Date of filing: **12.06.2015**

(51) Int Cl.:
***C07H 15/04** (2006.01)* ***C11D 1/12** (2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **23.06.2014 EP 14173379**

(71) Applicant: **BASF SE**
**67056 Ludwigshafen (DE)**

(72) Inventors:
- **Pottie, Laurence**
  **50823 Köln (DE)**
- **Max, Eva**
  **95448 Bayreuth (DE)**
- **Clasen, Frank**
  **40723 Hilden (DE)**
- **Schade, Christian**
  **67063 Ludwigshafen (DE)**

(74) Representative: **Gittinger, Andreas**
**BASF Personal Care und Nutrition GmbH**
**Henkelstrasse 67**
**40589 Düsseldorf (DE)**

# (54) DI(ALKYL-GLYCOSIDE) SULFOMETHYLSUCCINATE SURFACTANTS

(57) The present invention relates to an di(alkyl-glycoside) sulfomethylsuccinate having the formula $R^1$-O-$S_n$-$R^2$-$S_n$-O-$R^1$ wherein $R^1$ is an alkyl radical having 6 to 30 carbon atoms, S is a monosaccharide moiety, and $R^2$ is a sulfomethylsuccinate moiety. Furthermore the present invention relates to a process for making this di (alkyl-glycoside) sulfomethylsuccinate and to an di(alkyl-glycoside) itaconate which is a useful intermediate for use in this process. Furthermore the present invention relates to a cosmetic composition comprising the alkyl glycoside sulfomethylsuccinate.



Printed by Jouve, 75001 PARIS (FR)

## Description

The present invention relates to an di(alkyl-glycoside) sulfomethylsuccinate having the formula $R^1$-O-$S_n$-$R^2$-$S_n$-O-$R^1$ wherein $R^1$ is an alkyl radical having 6 to 30 carbon atoms, S is a monosaccharide moiety, and $R^2$ is a sulfomethylsuccinate moiety. Furthermore the present invention relates to a process for making this di(alkyl-glycoside) sulfomethylsuccinate and to an di(alkyl-glycoside) itaconate which is a useful intermediate for use in this process. Furthermore the present invention relates to a cosmetic composition comprising the alkyl glycoside sulfomethylsuccinate.

Alkyl glycosides are well known, mild, natural based, non-ionic surfactants. Many patent applications relate to alkyl glycosides. WO 90/03977 discloses a process for making alkyl glycosides.

Some work has already been done to further modify alkyl glycosides by esterification with diacids, mainly with the aim of obtaining anionic surfactants.

A representative example of such alkyl glycoside ester derivative is alkyl glycoside sulfosuccinate. This surfactant type is usually obtained in a two-step modification of alkyl glycosides. Firstly, the alkyl glycoside is reacted with maleic acid anhydride. Upon reaction the anhydride opens generating a carboxylic group. Then a second anionic group is generated by sulfonation of the alkyl glycoside maleate, using for instance sodium sulfite. This process is disclosed in US 7 087 571**.**

Alkyl glycoside sulfosuccinates are commercially available, e. g. under the trademark Eucarol® AGE SS from the company Lamberti.

EP 0 5105 65 A1 discloses the synthesis of di(alkyl glycoside) sulfosuccinates. The process for making them is basically the same as in the case of the monoesters apart from the molar ratio of the reagents. In this case the sulfonate group is the only anionic group, as the 2 carboxylic groups from the maleic acid are reacted into esters bonds.

The market demand for cosmetic ingredients based on renewable feedstock is constantly increasing. In this context alternatives to alkyl glycoside sulfosuccinates mono and diesters are desired because alkyl glycoside sulfosuccinates are not available based on renewable feedstock as their synthesis requires the use of petro-based maleic acid anhydride.

WO 2011/109047 **and** J. Ding, B. Song, C.Wang, J. Xu, Y. Wu, J. Surfact Deterg (2011) 14, 43-49**, Synthesis and Characterization of sodium Nonylphenol ethoxylate(10) sulfoitaconate esters; and** JP 58132092 A disclose alkyl sulofmethylsuccinate mono and diester surfactants based on fatty alcohols or fatty alcohol ethoxylates. These surfactants comprise a sulfonate group and they are made using itaconic acid anhydride. Itaconic acid anhydride can be obtained by dehydration of itaconic acid, a product obtained by fermentation of various natural feedstocks The resulting surfactants are called sulfoitaconates or sulofmethylsuccinates, since they only differ by one methyl group from the sulfosuccinate.

It is known that cosmetic formulations with lamellar structure have advantageous properties. They are for instance known to improve the rheological properties of the formulation, which is not only convenient for the user and giving a nice feeling but also helping to properly introduce some components in the formulation which would otherwise segregate if the viscosity was too low. Furthermore, vesicles formed by lamellar surfactants can be used to encapsulate actives. The various advantages of lamellar structures are broadly described in the literature like for instance in the review article from T. Engels and W. von Rybinski, J. Mater. Chem, 1998, 8(6), 1313-1320, Liquid crystalline surfactant phases in chemical applications**.**

The problem underlying the present invention is to provide a surfactant which can be used to make cosmetic formulations with a lamellar structure, and which can be made based on renewable feedstock.

This problem is solved by providing the di(alkyl-glycoside) sulfomethylsuccinate according to claim 1.

If M bears a charge z+ which is higher than 1+ then there is only 1/z part of this ion present to neutralize the negative charge of the -$SO_3^-$ group.

This di(alkyl-glycoside) sulfomethylsuccinate is a subject of the present invention.

According to the present invention the term alkyl glycoside means the reaction product of monosaccharides and fatty alcohols. A fatty alcohol is a linear, primary monoalkanol having 6 to 22 carbon atoms, optionally comprising up to 3 double bonds. A monosaccharide can be an aldose or a ketose, for example glucose, fructose, mannose, galactose, talose, gulose, allose, altrose, idose, arabinose, xylose, lyxose or ribose. The aldoses are preferably used by virtue of their better reactivity. Among the aldoses, glucose is particularly suitable because it is readily obtainable and available in industrial quantities. The alkyl glycosides produced with glucose are alkyl glucosides. Alkyl glycosides, depending on the specific process for making them, can comprise oligosaccharide moieties. Therefore, the terms alkyl oligoglycoside, alkyl polyglycoside, alkyl oligosaccharide and alkyl polysaccharide are used for alkyl glucosides in which an alkyl radical is attached to more than one glycose residue, i.e. to a poly- or oligosaccharide residue. These names are regarded as synonymous with one another. Accordingly, an alkyl monoglycoside comprises a monosaccharide moiety. Since mixtures are generally obtained in the acid-catalyzed reaction of sugars and fatty alcohols, the name alkyl glycoside is used in the following both for alkyl mono-glycosides and also for alkyl poly- or oligo-glycosides and, in particular, mixtures thereof. Alkyl glycosides have the formula $R^1$-O-$S_n$-H, wherein $R^1$ is an alkyl moiety derived from a

fatty alcohol which is bound to the mono- or oligo-saccharide moiety. It is assumed that this bond is an acetal bond, it is also conceivable that it is a hemiacetal bond or an ether bond. The degree of oligomerization of the saccharide moiety is denoted by n. Values between 1 and 5 (on average) are common. The average is a number average. The H in the formula is an H of an OH-group of the saccharide moiety. In the di(alkyl-glycoside) sulfomethylsuccinate according to the present invention this H is replaced by a sulfomethylsuccinate moiety which is bound both of its two COOH-groups to a saccharide moiety. It is assumed that these two bonds are ester bonds (i. e. the OH-groups are alcoholic OH-group), although it is also conceivable that the two COOH-groups of the sulfomethylsuccinate moiety are bound to OH-groups of saccharide moieties which are derived from their aldehyde or their ketone functionality.

In any case, there is no peroxo-group in the di(alkyl-glycoside) sulfomethylsuccinate having following formula (I): $R^1$-O-$S_n$-$R^2$-$S_n$-O-$R^1$. The alkyl glycosides having the formula $R^1$-O-$S_n$-H, wherein the H in this formula is an H of an OH-group of the saccharide moiety, react with two moles of itaconic acid under elimination of two moles of water so that the OH-groups together with the COOH-groups react to COO-groups. This means that the O-atoms of the COO-groups in formula (II) in claim 1 are bound to C-atoms of the $S_n$-moiety.

Instead of an alkyl glycoside derived from a fatty alcohol an alkyl glycoside derived from another mono-alcohol having a sufficiently long and therefore lipophilic alkyl chain can be used to make an di(alkyl-glycoside) sulfomethylsuccinate according to the present invention. <u>According to the present invention $R^1$ is</u> a linear or branched, saturated or unsaturated, primary, secondary or tertiary alkyl radical having 6 to 30 carbon atoms. In one embodiment of the present invention $R^1$ is a linear, primary alkyl radical having 6 to 22 carbon atoms, optionally comprising up to 3 double bonds, i. e. $R^1$ is derived from a fatty alcohol. In a more specific embodiment of the present invention $R^1$ is a linear, primary alkyl radical having 8 to 18 carbon atoms, optionally comprising up to 3 double bonds. More specifically $R^1$ is a linear, primary alkyl radical having 6 to 20 carbon atoms, optionally comprising up to 3 double bonds. More specifically $R^1$ is a linear, primary alkyl radical having 8 to 14 carbon atoms, optionally comprising up to 3 double bonds. More specifically $R^1$ is a saturated, linear, primary alkyl radical having 8 to 14 carbon atoms.

<u>According to the present invention S</u> is a monosaccharide moiety. In one embodiment of the present invention S is an aldose moiety. In a more specific embodiment of the present invention S is an aldose moiety having 6 carbon atoms. In a more specific embodiment of the present invention S is glucose moiety.

<u>According to the present invention n</u> is 1 to 5. In one embodiment of the present invention n is 1 to 1.5.

<u>According to the present invention $R^2$</u> is a sulfomethylsuccinate moiety according to formula (II) given in claim 1, <u>wherein M</u> is H or any cation. In one embodiment of the present invention M is selected from the group consisting of H, an alkali metal cation, $NH_4^+$ and mixtures thereof. In a more specific embodiment of the present invention M is selected from the group consisting of H, $Na^+$, $K^+$, $NH_4^+$ and mixtures thereof.

One embodiment of the present invention is the di(alkyl-glycoside) sulfomethylsuccinate according to the present invention, wherein $R^1$ is a saturated, linear, primary alkyl radical having 8 to 14 carbon atoms, S is glucose moiety, n is 1 to 1.5, and M is selected from the group consisting of H, $Na^+$, $K^+$, $NH_4^+$ and mixtures thereof.

Another subject of the present invention is a process for making the di(alkyl-glycoside) sulfomethylsuccinate according to the present invention comprising

a) reacting an alkyl glycoside $R^1$-O-$S_n$-H, wherein $R^1$, S and n have the meaning defined in any of claims 1 to 10, with itaconic acid, optionally in the presence of a catalyst, or with itaconic acid anhydride, optionally in the presence of a catalyst, so that an di(alkyl-glycoside) itaconate is obtained, and

b) reacting the di(alkyl-glycoside) itaconate with a sulfonating agent, preferably with a sulfite salt or with sulfurous acid, more preferably with sodium sulfite, so that the di(alkyl-glycoside) sulfomethylsuccinate according to any of claims 1 to 10 is obtained.

The catalyst that may be used in step a) described in the previous paragraph can be a catalyst that is appropriate for an esterification. Catalysts that can be used include, for example, acidic catalysts like alkyl sulfonic acid and in particular methane sulfonic acid, sulfuric acid or phosphoric acid, or metal ion based catalyst like zinc oxide, zinc acetate or zinc oxalate.

Another subject of the present invention is an di(alkyl-glycoside) itaconate which can be used as intermediate in the process for making the di(alkyl-glycoside) sulfomethylsuccinate according to the present invention. This di(alkyl-glycoside) itaconate is a di(alkyl-glycoside) itaconate having the following formula (I) given in claim 12, wherein $R^1$, S and n have the meaning defined in any of claims 1 to 10, and $R^3$ is a sulfomethylsuccinate moiety according to formula (III) given in claim 12.

Another subject of the present invention is a cosmetic composition, preferably a leave-on formulation such as a cream or a milk for face or body, or a rinse-off formulation such as a shampoo, shower gel or conditioner, comprising the di(alkyl-glycoside) sulfomethylsuccinate according to the present invention (preferably in an amount of from 0.01 to 30 % by weight, more preferably 0.5 to 20% by weight). In a more specific embodiment of the present invention this

cosmetic composition comprises an anionic surfactant different from the di(alkyl-glycoside) sulfomethylsuccinate (preferably in an amount of from 0.01 to 30 % by weight), and wherein this cosmetic formulation-preferably comprises a nonionic surfactant (preferably in an amount of from 0.01 to 30 % by weight).

The di(alkyl-glycoside) sulfomethylsuccinate according to the present invention can be used in any kind of formulation. Since it is mild to the skin it is particularly interesting for cosmetic or home care formulations. It can be used in mild foaming formulations such as shampoos or shower gels. In particular it can be used as primary or co-surfactants in formulations based, mostly or exclusively, on products based on renewable feedstock.

Another advantage of the present invention is that itaconic acid is based on renewable feedstock.

Itaconic acid itself is known to have antimicrobial activity. Therefore it seems not unlikely that the surfactant of the present invention may have antimicrobial activity, too.

## Examples

In the following % means % by weight unless specified differently.

### Synthesis of an alkyl glucoside sulfomethylsuccinate diester

495.0 g $C_{12/14}$-alkyl glucoside (Plantacare® 1200 UP: 50.8 % active matter, 600 mmol) were poured in a 2 L three-neck-flask, equipped with a stirrer, a distillation condenser and a nitrogen connection. After adjusting the pH to a value of 6.5 using 8.2 g of HCl (37 % aqueous solution), 50.4 g of a $C_{12/14}$-fatty alcohol (Lorol® $C_{12}$-$C_{14}$ Spezial, 260 mmol) were added. The mixture was then stirred under $N_2$ flow and heated to 120°C using an oil bath. The water contained in the mixture was distilled, at first under room atmosphere, then carefully applying vacuum (up to 20 mbar). After complete water removal 48.0 g of itaconic acid anhydride (429 mmol, China Jiangsu Int'l Economic and technical cooperation group, LTD / 98.8 %) were added and the reaction was continued under vacuum. The acid value was controlled each hour until a value below 40 $mg_{KOH}$/g was reached. The mixture was then cooled to 85°C. Then 42.0 g of $Na_2S_2O_5$ in form of an aqueous solution was added and the mixture was stirred for further 4 h. Finally, the excess sulfite was oxidized to sulfate using a 35 % aqueous $H_2O_2$ solution.

**Table 4:** Analysis of the alkyl glycoside sulfomethylsuccinate diester

| | **Content [%]** | **Method** |
|---|---|---|
| **$SO_3^{2-}$** | **0.2 %** | High Performance Ion Chromatography (HPIC) |
| **$SO_4^{2-}$** | **1.9 %** | |
| **Sulfomethylsuccinic acid** | **3.8 %** | High Performance Liquid Chromatography (HPLC) |
| **$C_{12/14}$-alkyl glucoside** | **19.2 %** | Gas Chromatography (GC) |
| **Alkyl glucoside conversion** | **41.0 %** | $\% \text{ conversion} = 100 - \left(\frac{\% \text{ not reacted alkyl glucoside x } 100}{\% \text{ primarily used alkyl glucoside}}\right)$ |
| **Water** | **48.5 %** | Karl Fischer (ISO 4317) |

### Properties of the alkyl glucoside sulfomethylsuccinate diester made

An emulsion according to the following recipe was prepared and analyzed by polarized light microscopy:

| | Ingredient | % by weight |
|---|---|---|
| I | Alkyl glucoside sulfomethylsuccinate diester | 1.12 |
| | Lanette® O (Cetearyl alcohol) | 5 |
| | Cetiol® LC (coco-caprylate/caprate) | 16 |
| II | Glycerol | 3 |
| | Water | 74.78 |

(continued)

| | Ingredient | % by weight |
|---|---|---|
| III | Euxyl® K100 (preservative) | 0.1 |
| Euxyl® K100 = benzyl alcohol (and) methylchloroisothiazolinone (and) methylisothiazolinone | | |

Phase I was heated and stirred until it was uniform. Phase II was heated separately to 80-85 °C and added to Phase I at 80°C while stirring. The emulsion was cooled down while stirring in such a way that it remained in continuous motion and that no air was incorporated. After gel formation at approx. 50°C the mixture was homogenized using an Ultra-Turrax mixer. While stirring the mixture was cooled down to 40 °C, then Phase III was added, still under stirring.

The mixture was then observed under a polarized light microscope. The anisotropic liquid crystal phase ("Malta's cross") indicates the presence of a lamellar structure, which is of advantage in cosmetic formulations.

## Claims

1. A di(alkyl-glycoside) sulfomethylsuccinate having the following formula (I),

$$R^1\text{-O-}S_n\text{-}R^2\text{-}S_n\text{-O-}R^1 \qquad \text{(I)}$$

wherein
$R^1$ is a linear or branched, saturated or unsaturated, primary, secondary or tertiary alkyl radical having 6 to 30 carbon atoms,
S is a monosaccharide moiety,
n is 1 to 5, and
$R^2$ is a sulfomethylsuccinate moiety according to formula (II)

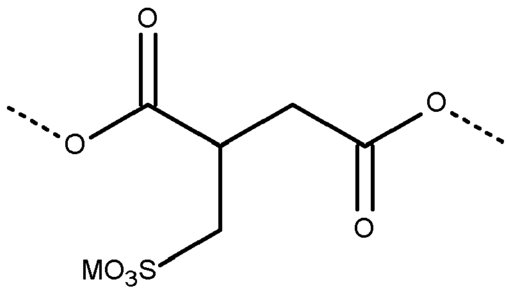

(II)

wherein
M is H or any cation.

2. The di(alkyl-glycoside) sulfomethylsuccinate according to claim 1, wherein $R^1$ is a linear, primary alkyl radical having 6 to 22 carbon atoms, optionally comprising up to 3 double bonds.

3. The di(alkyl-glycoside) sulfomethylsuccinate according to claim 1, wherein $R^1$ is a linear, primary alkyl radical having 8 to 18 carbon atoms, optionally comprising up to 3 double bonds,
preferably wherein $R^1$ is a linear, primary alkyl radical having 6 to 20 carbon atoms, optionally comprising up to 3 double bonds,
preferably wherein $R^1$ is a linear, primary alkyl radical having 8 to 14 carbon atoms,
optionally comprising up to 3 double bonds,
more preferably wherein $R^1$ is a saturated, linear, primary alkyl radical having 8 to 14 carbon atoms.

4. The di(alkyl-glycoside) sulfomethylsuccinate according to any of claims 1 to 3, wherein S is an aldose moiety.

**5.** The di(alkyl-glycoside) sulfomethylsuccinate according to claim 4, wherein S is an aldose moiety having 6 carbon atoms.

**6.** The di(alkyl-glycoside) sulfomethylsuccinate according to claim 4, wherein S is a glucose moiety.

**7.** The di(alkyl-glycoside) sulfomethylsuccinate according to any of claims 1 to 6, wherein n is 1 to 1.5.

**8.** The di(alkyl-glycoside) sulfomethylsuccinate according to any of claims 1 to 7, wherein M is selected from the group consisting of H, an alkali metal cation, $NH_4^+$ and mixtures thereof.

**9.** The di(alkyl-glycoside) sulfomethylsuccinate according to claim 8, wherein M is selected from the group consisting of H, $Na^+$, $K^+$, $NH_4^+$ and mixtures thereof.

**10.** The di(alkyl-glycoside) sulfomethylsuccinate according to claim 1, wherein
$R^1$ is a saturated, linear, primary alkyl radical having 8 to 14 carbon atoms,
S is a glucose moiety,
n is 1 to 1.5, and
M is selected from the group consisting of H, $Na^+$, $K^+$, $NH_4^+$ and mixtures thereof.

**11.** A process for making the di(alkyl-glycoside) sulfomethylsuccinate according to any of claims 1 to 10 comprising

a) reacting an alkyl glycoside $R^1$-O-$S_n$-H, wherein $R^1$, S and n have the meaning defined in any of claims 1 to 10, with itaconic acid, optionally in the presence of a catalyst, or with itaconic acid anhydride, optionally in the presence of a catalyst, so that an di(alkyl-glycoside) itaconate is obtained, and
b) reacting the di(alkyl-glycoside) itaconate with a sulfonating agent, preferably with a sulfite salt or with sulfurous acid, more preferably with sodium sulfite, so that the di(alkyl-glycoside) sulfomethylsuccinate according to any of claims 1 to 10 is obtained.

**12.** An di(alkyl-glycoside) itaconate having the following formula (I),

$$R^1\text{-O-}S_n\text{-}R^3\text{-}S_n\text{-O-}R^1 \qquad (I)$$

wherein $R^1$, S and n have the meaning defined in any of claims 1 to 10, and
$R^3$ is an itaconate moiety according to formula (III)

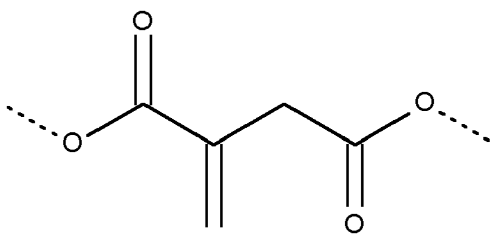

(III)

**13.** A cosmetic composition, preferably a shampoo or a shower gel, comprising the di(alkyl-glycoside) sulfomethylsuccinate according to any of claims 1 to 10, preferably in an amount of from 0.01 to 30 % by weight, more preferably 0.5 to 20% by weight.

**14.** The cosmetic composition according to claim 13, wherein this cosmetic composition is a shampoo or a shower gel, and wherein this shampoo or shower gel comprises an anionic surfactant different from the di(alkyl-glycoside) sulfomethylsuccinate (preferably in an amount of from 0.01 to 30 % by weight), and wherein this shampoo or shower gel preferably comprises a nonionic surfactant (preferably in an amount of from 0.01 to 30 % by weight).

Europäisches Patentamt
European Patent Office
Office européen des brevets

# EUROPEAN SEARCH REPORT

Application Number
EP 15 17 1792

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2011/109047 A2 (STEPAN CO [US]; BROWN AARON MICHAEL; DONG XUE MIN) 9 September 2011 (2011-09-09) * the whole document * ----- | 1-14 | INV. C07H15/04 C11D1/12 |
| A,D | FR 2 785 794 A1 (OREAL [FR]) 19 May 2000 (2000-05-19) * the whole document * ----- | 1-14 | |
| A | WO 97/42299 A1 (HENKEL CORP [US]) 13 November 1997 (1997-11-13) * the whole document * ----- | 1-14 | |

TECHNICAL FIELDS SEARCHED (IPC)
C07H
C11D

The present search report has been drawn up for all claims

1

| Place of search | Date of completion of the search | Examiner |
|---|---|---|
| Munich | 14 August 2015 | Klein, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 17 1792

This annex lists the patent family members relating to the patent documents cited in the above-mentioned European search report.
The members are as contained in the European Patent Office EDP file on
The European Patent Office is in no way liable for these particulars which are merely given for the purpose of information.

14-08-2015

| Patent document cited in search report | Publication date | Patent family member(s) | Publication date |
|---|---|---|---|
| WO 2011109047 A2 | 09-09-2011 | AU 2010347222 A1 | 24-05-2012 |
| | | CA 2779644 A1 | 09-09-2011 |
| | | CN 102712563 A | 03-10-2012 |
| | | CO 6541626 A2 | 16-10-2012 |
| | | EP 2496545 A2 | 12-09-2012 |
| | | US 2012270764 A1 | 25-10-2012 |
| | | WO 2011109047 A2 | 09-09-2011 |
| FR 2785794 A1 | 19-05-2000 | AT 215813 T | 15-04-2002 |
| | | AU 6094399 A | 05-06-2000 |
| | | DE 69901228 D1 | 16-05-2002 |
| | | DE 69901228 T2 | 02-10-2002 |
| | | EP 1047403 A1 | 02-11-2000 |
| | | ES 2176028 T3 | 16-11-2002 |
| | | FR 2785794 A1 | 19-05-2000 |
| | | WO 0028965 A1 | 25-05-2000 |
| WO 9742299 A1 | 13-11-1997 | AU 2668297 A | 26-11-1997 |
| | | BR 9708918 A | 03-08-1999 |
| | | CA 2251376 A1 | 13-11-1997 |
| | | EP 0904346 A1 | 31-03-1999 |
| | | JP 2000509713 A | 02-08-2000 |
| | | KR 20000010805 A | 25-02-2000 |
| | | US 5908928 A | 01-06-1999 |
| | | WO 9742299 A1 | 13-11-1997 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9003977 A
- US 7087571 B
- EP 0510565 A1
- WO 2011109047 A
- JP 58132092 A


### Non-patent literature cited in the description


- **J. DING ; B. SONG ; C.WANG ; J. XU ; Y. WU.** *J. Surfact Deterg,* 2011, vol. 14, 43-49
- **T. ENGELS ; W. VON RYBINSKI.** Liquid crystalline surfactant phases in chemical applications. *J. Mater. Chem,* 1998, vol. 8 (6), 1313-1320